# EUROPÄISCHE PATENTSCHRIFT

(11) **EP 2 851 131 B1**
(45) Veröffentlichungstag und Bekanntmachung des Hinweises auf die Patenterteilung: **26.04.2017**
(21) Anmeldenummer: 14176126.2
(22) Anmeldetag: 08.07.2014
(51) Int. Cl.: B05B 11/00, B05B 1/34, A61M 11/00, A45D 34/00, A61M 31/00

(54) **Abgabevorrichtung für Fluide aus einem Fluidbehälter**
Dispenser for fluids from a fluid container
Dispositif de dépôt pour fluide provenant d'un récipient de fluide

(30) Priorität: 19.09.2013 DE 102013218802
(43) Veröffentlichungstag der Anmeldung: 25.03.2015
(73) Patentinhaber: Aero Pump GmbH, 65239 Hochheim/Main (DE)
(72) Erfinder: Mersmann, Andreas, 60326 Frankfurt (DE)
(74) Vertreter: Knoblauch, Andreas

(56) Entgegenhaltungen:
- EP-A1- 1 570 910
- EP-A1- 2 607 266
- WO-A1-98/09732
- WO-A1-2011/150021
- US-A1- 2008 041 889

## Beschreibung

Die Erfindung betrifft eine Abgabevorrichtung für Fluide aus einem Fluidbehälter umfassend einen Befestigungsabschnitt, einen ersten Blockierabschnitt und einen Betätigungsabschnitt, wobei die Abgabe des Fluides durch Betätigung des Betätigungsabschnitts erfolgt, und wobei die Betätigung des Betätigungsabschnitts durch ein Verdrehen des ersten Blockierabschnitts gegenüber dem Befestigungsabschnitt blockierbar ist.

Derartige Abgabevorrichtungen dienen beispielsweise der Abgabe von Fluiden, wie medizinischen Wirkstofflösungen, in Nase, Mund oder Augen oder der Abgabe von Kosmetikprodukten. Mit Hilfe des Befestigungsabschnitts erfolgt eine Befestigung der Abgabevorrichtung am Fluidbehälter.

Durch ein manuelles Betätigen des Betätigungsabschnitts wird dabei üblicherweise eine vorbestimmte Menge des Fluides aus dem Fluidbehälter gefördert und zu einer Ausgabeöffnung transportiert.

Eine Abgabevorrichtung der eingangs genannten Art ist beispielsweise aus DE 602 11 855 T2 bekannt. Die dortige Abgabevorrichtung umfasst einen Befestigungsabschnitt, einen Betätigungsabschnitt sowie eine Sperreinrichtung. Mit Hilfe der Sperreinrichtung lässt sich die Betätigung des Betätigungsabschnitts durch ein Verdrehen des Betätigungsabschnitts gegenüber dem Befestigungsabschnitt verhindern.

WO 2011/150021 A1 zeigt eine Abgabevorrichtung umfassend einen Betätigungsabschnitt sowie einen Befestigungsabschnitt, mit dem die Abgabevorrichtung an einem Fluidbehälter befestigt ist. Der Betätigungsabschnitt ist gegenüber dem Befestigungsabschnitt verdrehbar, um die Abgabevorrichtung blockieren zu können. Der Betätigungsabschnitt sowie der Befestigungsabschnitt weisen dazu jeweils eine umlaufende Kontur auf, die in einer Freigabeposition ineinander greifen und somit eine Betätigung der Abgabevorrichtung ermöglichen. Der Befestigungsabschnitt kann auch gegenüber dem Fluidbehälter verdrehbar ausgebildet sein, wodurch sich jedoch keine zusätzliche Blockadefunktion ergibt.

EP 1 570 910 A1 zeigt eine manuell betätigbare Abgabevorrichtung mit einem Betätigungsabschnitt und einem Befestigungsabschnitt. Durch ein Verdrehen des Betätigungsabschnitts gegenüber dem Befestigungsabschnitt lässt sich eine Blockade bzw. Freigabe der Betätigung des Betätigungsabschnitts erreichen. Die Abgabevorrichtung umfasst weiterhin eine Kappe, die gegenüber dem Betätigungsabschnitt verdrehbar ausgebildet ist. Durch ein Verdrehen der Kappe gegenüber dem Betätigungsabschnitt lässt sich ein Fluidausgang verschließen, um eine Abgabe des Fluids aus der Abgabevorrichtung zu verhindern.

Aus WO 98/09732, die den Oberbegriff des Anspruchs 1 offenbart, ist eine weitere Abgabevorrichtung für Fluide mit einer Sperrvorrichtung bekannt. Die dortige Abgabevorrichtung umfasst einen drehbaren Betätigungsabschnitt mit einem oder mehreren axial verlaufenden Arretierstiften. In einem Gehäuse sind unterhalb des Betätigungsabschnitts drei drehbare Scheiben angeordnet. Die Scheiben verfügen über Löcher. In einer entsperrten Relativstellung des Betätigungsabschnitts und der Scheiben können die Arretierstifte in die Löcher eindringen und der Betätigungsabschnitt betätigt werden. Die Scheiben verfügen über Klinkenzähne, durch die der Betätigungsabschnitt über die obere Scheibe indirekt die mittlere Scheibe mitdrehen kann, sofern die Klinkenzähne der oberen Scheibe an die Klinkenzähne der mittleren Scheibe anstoßen. Ein Entsperren erfolgt durch ein mehrfaches Verdrehen des Betätigungsabschnitts um vordefinierte Drehwinkel in bzw. gegen den Uhrzeigersinn, wodurch die Scheiben in eine entsperrte Position gebracht werden.

Abgabevorrichtungen dieser Art ermöglichen zwar im Prinzip eine ungewollte Betätigung der Abgabevorrichtung beim Transport und insbesondere vor dem Eintreffen beim Endverbraucher zu vermeiden. Allerdings ist ein ungewolltes Entsperren der Abgabevorrichtung nach wie vor möglich, da lediglich eine gesperrte und eine entsperrte Einstellung der Sperrvorrichtung vorgesehen ist.

Ein Entsperren wird üblicherweise durch ein gleichzeitiges Betätigen und Drehen des Betätigungsabschnitts erreicht. Dies hat den zusätzlichen Nachteil, dass in einigen Fällen beim Entsperren der Abgabevorrichtung bereits ungewollt Fluid abgegeben werden kann.

Weiterhin ist der Entsperrvorgang der bekannten Abgabevorrichtungen verhältnismäßig einfach, so dass die Sperrvorrichtung auch von einem Kleinkind durch Ausprobieren leicht überwunden werden kann. In diesem Fall besteht, insbesondere wenn der Fluidbehälter ein Medikament enthält, eine Vergiftungsgefahr für das Kleinkind.

Der Erfindung liegt daher die Aufgabe zugrunde eine Abgabevorrichtung für Fluide aus einem Fluidbehälter zur Verfügung zu stellen, die zuverlässig blockierbar ist.

Erfindungsgemäß wird diese Aufgabe bei einer Abgabevorrichtung der eingangs genannten Art dadurch gelöst, dass die Abgabevorrichtung mindestens zwei Blockierabschnitte aufweist, wobei die Betätigung der Abgabevorrichtung auch durch ein Verdrehen des zweiten Blockierabschnitts gegenüber dem Befestigungsabschnitt blockierbar ist, wobei die Abgabevorrichtung nur in einer relativen Position von erstem Blockierabschnitt, zweitem Blockierabschnitt und Befestigungsabschnitt betätigt werden kann.

Eine Betätigung der Abgabevorrichtung lässt sich also nunmehr sowohl durch ein Verdrehen des ersten Blockierabschnitts gegenüber dem Befestigungsabschnitt als auch durch ein Verdrehen des zweiten Blockierabschnitts gegenüber dem Befestigungsabschnitt blockieren. Hierbei genügt es bereits für eine Blockierung der Abgabevorrichtung, dass entweder der erste Blockierabschnitt oder der zweite Blockierabschnitt gegenüber dem Befestigungsabschnitt verdreht ist. Zum Sperren bzw. Entsperren der Abgabevorrichtung ist es dabei nicht mehr nötig gleichzeitig den Betätigungsabschnitt zu betätigen, wodurch eine ungewollte Abgabe von Fluid beim Sperren bzw. Entsperren vermieden wird. Weiterhin ergibt sich auch eine Abgabevorrichtung mit einer effektiven Kindersicherung, da sich sowohl der erste Blockierabschnitt als auch der zweite Blockierabschnitt gegenüber den Befestigungsabschnitt in einer korrekten Drehposition befinden muss, um eine Betätigung der Abgabevorrichtung zu ermöglichen. So lässt es sich problemlos realisieren eine Vielzahl an blockierten relativen Drehpositionen von erstem Blockierabschnitt bzw. zweitem Blockierabschnitt gegenüber dem Befestigungsabschnitt einzurichten. Vorzugsweise erlaubt sowohl der erste Blockierabschnitt als auch der zweite Blockierabschnitt jeweils nur in einer relativen Drehposition zum Befestigungsabschnitt eine Betätigung der Abgabevorrichtung. Die Abgabevorrichtung kann auch mehr als zwei, beispielsweise drei oder vier, Blockierabschnitte umfassen, falls eine besonders hohe Sicherheit der Abgabevorrichtung gewünscht ist.

In einer bevorzugten Ausführungsform ist der erste Blockierabschnitt drehfest mit dem Betätigungsabschnitt verbunden. Der erste Blockierabschnitt kann also beispielsweise einstückig mit dem Betätigungsabschnitt in einem gemeinsamen Bauteil angeordnet sein. Alternativ können der erste Blockierabschnitt und der Betätigungsabschnitt auch in separaten, gegeneinander verdrehbaren Bauteilen angeordnet sein.

Vorzugsweise weist der erste Blockierabschnitt relativ zum Befestigungsabschnitt mindestens eine diskrete Freigabeposition und mindestens zwei diskrete Blockierpositionen auf. Unter einer diskreten Position ist dabei eine vordefinierte Drehstellung zu verstehen, in die sich der erste Blockierabschnitt relativ zum Befestigungsabschnitt drehen lässt, und dann beispielsweise einrastet. Hierbei ist es bevorzugt, wenn zum Wechseln von einer diskreten Position in eine andere diskrete Position ein kleiner mechanischer Widerstand überwunden werden muss, wodurch ein ungewolltes Verdrehen des ersten Blockierabschnitts gegenüber dem Befestigungsabschnitt vermieden werden kann.

Es ist von Vorteil, wenn der zweite Blockierabschnitt relativ zum Befestigungsabschnitt mindestens eine diskrete Freigabeposition und mindestens zwei diskrete Blockierpositionen aufweist. Besonders bevorzugt ist es dabei, wenn sowohl der erste Blockierabschnitt als auch der zweite Blockierabschnitt relativ zum Befestigungsabschnitt jeweils mindestens neun diskrete Blockierpositionen aufweisen. In diesem Fall ergeben sich bereits insgesamt mindestens 100 relative Einstellpositionen von erstem Blockierabschnitt, zweitem Blockierabschnitt und Befestigungsabschnitt, wobei die Abgabevorrichtung in mindestens 99 Einstellungen blockiert ist. In mindestens einer diskreten Freigabeposition ist aber stets eine Betätigung möglich. Ein Betätigen der Abgabevorrichtung ohne ein Verständnis des Mechanismus durch reines Ausprobieren ist dann praktisch ausgeschlossen.

Weiterhin ist es von Vorteil, wenn der zweite Blockierabschnitt in Betätigungsrichtung zwischen dem ersten Blockierabschnitt und dem Befestigungsabschnitt angeordnet und relativ zum ersten Blockierabschnitt verdrehbar ist. Hierdurch ergibt sich ein vergleichsweise einfacher Aufbau der Abgabevorrichtung. Vorzugsweise ist dabei der zweite Blockierabschnitt drehbar mit dem ersten Blockierabschnitt verbunden, insbesondere verrastet. Hierdurch lässt sich sicherstellen, dass zur Betätigung der Abgabevorrichtung sowohl der erste Blockierabschnitt als auch der zweite Blockierabschnitt in Betätigungsrichtung relativ um Befestigungsabschnitt verschoben werden müssen.

Es ist von Vorteil, wenn auf einer Außenseite des ersten Blockierabschnitts, des zweiten Blockierabschnitts und des Befestigungsabschnitts jeweils mindestens eine Markierung angeordnet ist. Vorzugsweise sind diese Markierungen jeweils an einer radialen Außenseite des ersten Blockierabschnitts, des zweiten Blockierabschnitts und des Befestigungsabschnitts angeordnet. Beispielsweise können die Markierungen auf den drei genannten Abschnitten so angeordnet werden, dass die Abgabevorrichtung nur dann betätigt werden kann, wenn alle drei Markierungen sich in Betätigungsrichtung hintereinander befinden bzw. auf denselben Drehwinkel relativ zu einer gemeinsamen Drehachse gedreht sind. Eine solche Markierung kann beispielsweise in Form einer kleinen radial vorstehenden Spitze vorliegen, wodurch die Abgabevorrichtung auch von Personen mit verringerter Sehkraft bedient werden kann. Alternativ ist es auch vorstellbar, dass auf mindestens zwei der drei verdrehbaren Abschnitte Zahlenmarkierungen angebracht sind, und die Abgabevorrichtung nur bei Einstellung einer bestimmten Zahlenkombination betätigt werden kann. In diesem Fall bilden der erste Blockierabschnitt, der zweite Blockierabschnitt und der Befestigungsabschnitt also gemeinsam ein Zahlenschloss. Eine solche Ausführungsform ist beispielsweise bei für Kleinkinder besonders gefährlichen Medikamenten vorstellbar.

Es ist vorteilhaft, wenn zwischen dem ersten Blockierabschnitt, dem zweiten Blockierabschnitt und dem Befestigungsabschnitt mindestens eine Sperrvorrichtung angeordnet ist. Die mindestens eine Sperrvorrichtung kann dann beispielsweise ein axiales Verschieben von erstem Blockierabschnitt und zweitem Blockierabschnitt in Betätigungsrichtung verhindern, wenn der erste Blockierabschnitt und der zweite Blockierabschnitt nicht in die korrekten Relativpositionen jeweils gegenüber dem Befestigungsabschnitt gedreht sind.

Weiterhin ist es von Vorteil, wenn die mindestens eine Sperrvorrichtung mindestens eine Aussparung und mindestens einen Vorsprung aufweist. Die mindestens eine Aussparung und der mindestens eine Vorsprung können vorzugsweise im unbetätigten Zustand der Abgabevorrichtung in Betätigungsrichtung axial versetzt angeordnet sein, können jedoch einen übereinstimmenden radialen Abstand zu einer gemeinsamen Drehachse aufweisen.

Vorzugsweise blockiert die mindestens eine Sperrvorrichtung die Betätigung des Betätigungsabschnitts, es sei denn, die mindestens eine Aussparung und der mindestens eine Vorsprung sind relativ zu einer gemeinsamen Drehachse von erstem Blockierabschnitt, zweitem Blockierabschnitt und Befestigungsabschnitt auf denselben Drehwinkel gedreht. Hierbei ist es besonders bevorzugt, wenn die mindestens eine Aussparung und der mindestens eine Vorsprung jeweils in Radialrichtung mit einer Markierung an einer radialen Außenseite übereinstimmen. In diesem Fall ist es für den Endverbraucher besonders einfach, die Abgabevorrichtung zu entsperren.

Weiterhin ist es von Vorteil, wenn der Vorsprung in einem unbetätigten Zustand der Abgabevorrichtung an einer Außenseite der Abgabevorrichtung angeordnet ist. In diesem Fall kann der Vorsprung vom Endverbraucher problemlos erkannt werden, wodurch der Vorsprung gleichzeitig die Funktion einer der Markierungen übernehmen kann.

Vorzugsweise weisen der erste Blockierabschnitt und der zweite Blockierabschnitt mindestens je eine Aussparung auf und der Befestigungsabschnitt weist mindestens einen Vorsprung auf. Somit lässt sich beispielsweise realisieren, dass die Abgabevorrichtung nur dann betätigt werden kann, wenn sowohl die beiden Aussparungen als auch der eine Vorsprung relativ zu einer gemeinsamen Drehachse auf denselben Drehwinkel gedreht sind. Dann kann beim Betätigen des Betätigungsabschnitts der Vorsprung in die beiden Aussparungen von Betätigungsabschnitt und Blockierabschnitt eingeschoben werden.

Weiterhin ist es von Vorteil, wenn der erste Blockierabschnitt und / oder der zweite Blockierabschnitt einen Blockierring umfasst, der mit dem Betätigungsabschnitt drehbar verbunden ist. Vorzugsweise ist der Blockierring hierbei mit dem Betätigungsabschnitt drehbar verrastet, wodurch sich ein stabiler und kompakter Aufbau ergibt. Es können aber auch beide Blockierabschnitte je einen Blockierring aufweisen, wobei dann vorzugsweise der Blockierring des ersten Blockierabschnitts mit dem Betätigungsabschnitt drehbar verrastet ist. In letzterem Fall können auch beide Blockierringe drehbar miteinander verrastet sein.

Es ist von Vorteil, wenn die Abgabevorrichtung einen Führungsring aufweist, der in Betätigungsrichtung gegenüber dem Befestigungsabschnitt verschiebbar ist. Der Führungsring erlaubt eine kontrollierte Relativbewegung von erstem Blockierabschnitt, zweitem Blockierabschnitt und Befestigungsabschnitt in Betätigungsrichtung. Insbesondere kann der Führungsring auch die auf die Abgabevorrichtung wirkenden Kräfte während des Betätigungsvorgangs abfedern. Eine Beschädigung der empfindlicheren inneren Bauteile der Abgabevorrichtung lässt sich somit vermeiden. Weiterhin kann der der Führungsring auch als Sichtschutz wirken, so dass die Freigabepositionen der Blockierabschnitte nicht von außen einsehbar sind. Letzteres ist vor allem dann von Vorteil, wenn die Blockierabschnitte mit dem Befestigungsabschnitt ein Zahlenschloss bilden.

Vorzugsweise ist der Führungsring gegenüber dem Befestigungsabschnitt drehfest angeordnet. Hierzu kann der Führungsring beispielsweise in Umfangsrichtung eine Lücke aufweisen, in die ein Vorsprung des Befestigungsabschnitts eingreift. Gleichzeitig kann der Führungsring an einer axialen Stirnseite am ersten Blockierabschnitt und / oder am zweiten Blockierabschnitt anliegen. Hierdurch wird sichergestellt, dass der Führungsabschnitt bei einem Betätigungsvorgang gemeinsam mit dem ersten Blockierabschnitt und / oder dem zweiten Blockierabschnitt in Betätigungsrichtung verschoben wird und somit eine kontrollierte Bewegung der drei verschiedenen Abschnitte relativ zueinander erfolgt.

Die Erfindung wird im Folgenden anhand eines bevorzugten Ausführungsbeispiels in Verbindung mit den Zeichnungen beschrieben. Hierin zeigen:
- Fig. 1: eine Abgabevorrichtung für Fluide entsprechend der Erfindung in einer unbetätigten Position, wobei sich sowohl der erste Blockierabschnitt als auch der zweite Blockierabschnitt in einer Freigabeposition befinden,
- Fig. 2: die Abgabevorrichtung entsprechend Fig. 1 entlang dem Querschnitt B-B,
- Fig. 3: die Abgabevorrichtung entsprechend Fig. 1 entlang dem Querschnitt C-C,
- Fig. 4: einen Längsschnitt einer Abgabevorrichtung entsprechend der Erfindung, wobei sich der erste Blockierabschnitt in einer Blockadeposition und der zweite Blockierabschnitt in einer Freigabeposition befindet,
- Fig. 5: ein Querschnitt der Abgabevorrichtung entsprechend Fig. 4 entlang dem Querschnitt B-B,
- Fig. 6: ein Querschnitt der Abgabevorrichtung entsprechend Fig. 4 entlang dem Querschnitt C-C,
- Fig. 7: einen weiteren Längsschnitt einer Abgabevorrichtung entsprechend der Erfindung, wobei sich sowohl der erste Blockierabschnitt als auch der zweite Blockierabschnitt in einer Blockadeposition befinden,
- Fig. 8: die Abgabevorrichtung entsprechend Fig. 7 entlang dem Querschnitt B-B,
- Fig. 9: die Abgabevorrichtung entsprechend Fig. 7 entlang dem Querschnitt C-C.

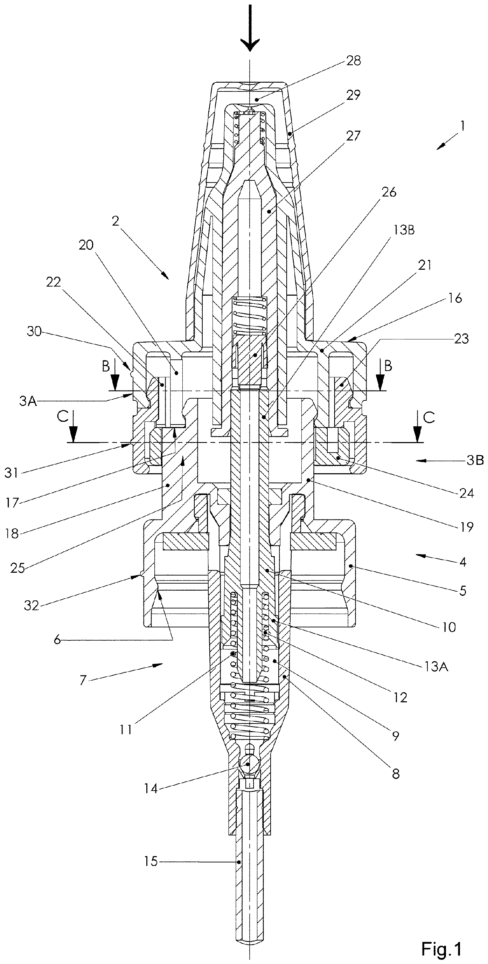

Die Fig. 1 bis 3 zeigen eine erste Einstellung der Abgabevorrichtung 1. Die Abgabevorrichtung 1 umfasst einen Betätigungsabschnitt 2, einen ersten Blockierabschnitt 3A, einen zweiten Blockierabschnitt 3B sowie einen Befestigungsabschnitt 4.

Im vorliegenden Ausführungsbeispiel ist der erste Blockierabschnitt 3A einstückig mit dem Betätigungsabschnitt 2 in einem Bauteil angeordnet. Hierbei ist der Betätigungsabschnitt 2 mit dem Befestigungsabschnitt 4 verrastet und der Betätigungsabschnitt 2 mit dem zweiten Blockierabschnitt 3B verrastet.

Es sind jedoch auch andere Ausführungsformen vorstellbar, wobei lediglich sichergestellt sein sollte, dass der erste Blockierabschnitt sowie der zweite Blockierabschnitt jeweils gegenüber dem Befestigungsabschnitt drehbar angeordnet sind. Beispielsweise können zwei oder mehr Blockierabschnitte als separate, gegenüber Betätigungsabschnitt und Befestigungsabschnitt verdrehbare Bauteile ausgebildet sein. Es können auch drei oder vier Blockierabschnitte, beispielsweise als drehbare Ringe, zwischen Betätigungsabschnitt und Befestigungsabschnitt angeordnet sein.

Der Befestigungsabschnitt 4 weist im vorliegenden Fall einen zylinderförmigen Aufsteckabschnitt 5 auf. Der Aufsteckabschnitt 5 weist eine radial nach innen gerichtet Halterippe 6 auf, mit der er auf einem Fluidbehälter einrasten kann. Hier ist alternativ auch ein Schraubverschluss oder ein vergleichbarer Befestigungsmechanismus vorstellbar.

Die Abgabevorrichtung 1 umfasst weiterhin eine Druckkammer 7 mit einem Druckkammergehäuse 8. Das Druckkammergehäuse 8 weist einen zylinderförmigen Abschnitt auf, der koaxial entlang der Betätigungsachse der Abgabevorrichtung 1 angeordnet ist. In einen Druckkammerinnenraum 9 der Druckkammer 7 greift ein Kolben 10 ein.

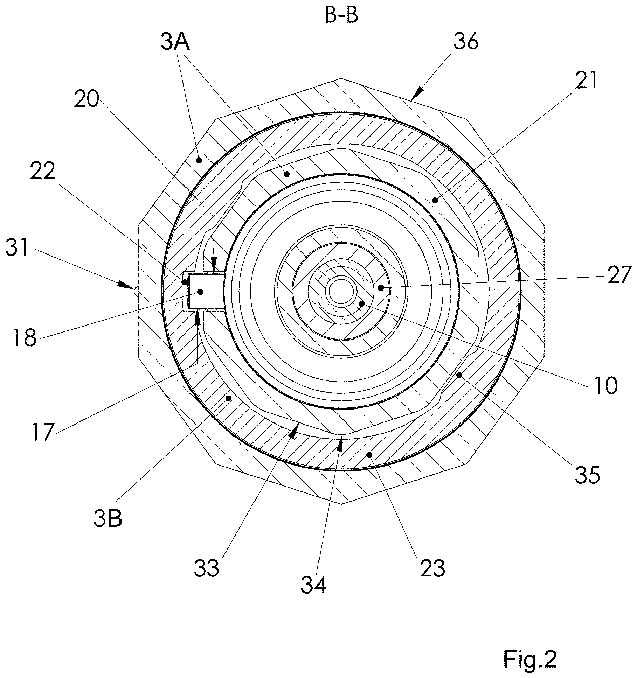

Die Abgabevorrichtung 1 ist in Fig. 1 (sowie in den nachfolgenden Fig. 2 bis 9) in einer Neutralposition dargestellt, das heißt, die Abgabevorrichtung ist in einem unbetätigten Zustand dargestellt. Zwischen dem Kolben 10 und dem Druckkammergehäuse 8 ist eine Feder 11 angeordnet. Die Feder 11 liegt an einem axialen Ende am Druckkammergehäuse 8 an. An ihrem gegenüberliegenden axialen Ende greift die Feder 11 in eine stirnseitige Nut 12 des Kolbens 10 ein.

Der Kolben 10 ist im Wesentlichen rohrförmig ausgebildet und weist in seinen Endabschnitten Öffnungen auf. Mit seinem ersten Endabschnitt 13A greift der Kolben 10 in die Druckkammer 7 ein. Mit seinem zweiten Endabschnitt 13B greift der Kolben 10 in den Betätigungsabschnitt 2 ein.

Das Druckkammergehäuse 8 umfasst außerdem ein Ventil 14, das im vorliegenden Fall als Kugelventil ausgestaltet ist. Im geöffneten Zustand erlaubt das Ventil 14 das Einströmen eines Fluides aus dem Fluidbehälter (nicht dargestellt). In das Druckkammergehäuse 8 ist weiterhin ein Stutzen 15 eingeschoben, der ein Ansaugen des Fluides vom Boden des Fluidbehälters ermöglicht, ohne dass sich das Druckkammergehäuse 8 selbst bis dorthin erstrecken muss. Dadurch wird sichergestellt, dass möglichst wenig des oftmals teuren Fluides verschwendet wird und ein mit der Abgabevorrichtung 1 zu verbindender Fluidbehälter möglichst vollständig geleert werden kann.

Wird die Abgabevorrichtung nun betätigt, so wird ein Teil des im Druckkammerinnenraum 9 enthaltenden Fluides abgegeben. Bei der Rückkehr in die Neutralposition entsteht daher im Druckkammerinnenraum 9 ein Unterdruck. Durch den Unterdruck im Druckkammerinnenraum 9 öffnet sich nun das Ventil 14 und neues Fluid wird aus dem Fluidbehälter in den Druckkammerinnenraum 9 angesaugt.

Der Betätigungsabschnitt 2 weist einen Betätigungsvorsprung 16 in Form einer umlaufenden Stufe auf. Zur Betätigung der Abgabevorrichtung 1 übt der Verbraucher nun einen Druck auf den Betätigungsvorsprung 16 in Betätigungsrichtung aus. Die Betätigungsrichtung ist in Fig. 1 durch einen entlang der zentralen Achse verlaufenden Pfeil angedeutet.

In Fig. 1 bis 3 ist sowohl der erste Blockierabschnitt 3A als auch der zweite Blockierabschnitt 3B in einer Freigabeposition. Dies ist in der vorliegenden Ausführungsform dadurch realisiert, dass zwischen dem Betätigungsabschnitt 2 und dem Befestigungsabschnitt 4 eine Sperrvorrichtung 17 angeordnet ist. Die Sperrvorrichtung 17 umfasst hier einen im Befestigungsabschnitt 4 angeordneten Vorsprung 18. Der Vorsprung 18 ist als radial nach außen verlaufende Erweiterung eines Innenzylinders 19 ausgeführt. Der Innenzylinder 19 ist dabei Bestandteil des Befestigungsabschnitts 4.

Die Sperrvorrichtung 17 weist weiterhin eine Aussparung 20 auf, die in einer zylinderförmigen Innenhülse 21 des ersten Blockierabschnitts 3A angeordnet ist. Die Sperrvorrichtung 17 umfasst außerdem eine Aussparung 22 die in einem Blockierring 23 des zweiten Blockierabschnitts 3B angeordnet ist. Die Aussparung 22 ist an einer radialen Innenseite des Blockierrings 23 angeordnet.

In der vorliegenden Einstellung der Abgabevorrichtung 1 befinden sich sowohl der erste Blockierabschnitt 3A als auch der zweite Blockierabschnitt 3B in einer Freigabeposition, da der Vorsprung 18 sowie die Aussparungen 20, 22 auf denselben Drehwinkel relativ zu einer gemeinsamen Drehachse der Abgabevorrichtung 1 gedreht sind. Die Drehachse stimmt hier mit der Zylinderachse der Abgabevorrichtung 1 über ein, die sich in Betätigungsrichtung erstreckt.

Übt der Verbraucher nun einen Druck auf den Betätigungsvorsprung 16 aus, so bewegen sich der Betätigungsabschnitt 2 mit dem ersten Blockierabschnitt 3A und der zweite Blockierabschnitt 3B auf den Befestigungsabschnitt 4 zu. Dabei wird der Innenzylinder 19 in den Betätigungsabschnitt 2 eingeschoben. Gleichzeitig dringt der Vorsprung 18 in die Aussparungen 20, 22 ein.

Um sicherzustellen, dass dieser Verschiebungsvorgang möglichst gleichmäßig von statten geht, ist zwischen dem Befestigungsabschnitt 4 und dem zweiten Blockierabschnitt 3B ein Führungsring 24 angeordnet. Der Führungsring 24 weist eine Lücke 25 auf, in die der Vorsprung 18 sowohl in der Neutralposition als auch während des Betätigungsvorgangs eingreift. Der Führungsring 24 liegt weiterhin an einer Stirnseite in Betätigungsrichtung am ersten Blockierabschnitt 3A sowie am zweiten Blockierabschnitt 3B an. Insgesamt wird hiermit sichergestellt, dass der erste Blockierabschnitt 3A und der zweite Blockierabschnitt 3B kontrolliert gegenüber dem Befestigungsabschnitt 4 verschiebbar sind. Gleichzeitig verdeckt der Führungsring 24 auch die Position der Aussparungen 20, 22 und wirkt somit als Sichtschutz.

Durch das Betätigen des Betätigungsabschnitts 2 verringert sich nun das Volumen des Druckkammerinnenraums 9, wodurch sich das Ventil 14 schließt und es zu einer Druckerhöhung kommt. In Folge der Druckerhöhung im Druckkammerinnenraum 9 öffnet sich ein Zwischenventil 26. Das Zwischenventil 26 ist dabei in einem Innenbauteil 27 angeordnet. Das Innenbauteil 27 ist radial innerhalb des Betätigungsabschnitts 2 angeordnet. Durch die Öffnung des Zwischenventils 26 wird eine nicht dargestellte radiale Bohrung des Innenbauteils 27 freigegeben. In Folge dessen wird das Fluid zwischen einer Innenseite des Betätigungsabschnitts 2 und einer Außenseite des Innenbauteils 27 bis zum Fluidausgang 28 geleitet.

Nach Abgabe des Fluides durch den Fluidausgang 28 bewegen sich der Betätigungsabschnitt 2 und der zweite Blockierabschnitt 3B wieder in ihre Neutralposition. Dabei entsteht wiederrum ein Unterdruck im Druckkammerinnenraum 9, wodurch sich das Ventil 14 öffnet und neues Fluid in die Druckkammer 7 nachströmen kann.

Die Abgabevorrichtung 1 verfügt hier auch über eine Schutzkappe 29, die vor einer Betätigung durch den Verbraucher abzunehmen ist.

In der vorliegenden Ausführungsform ist die Position der Aussparung 20 durch eine an einer radialen Außenseite des ersten Blockierabschnitts 3A angeordnete Markierung 30 für den Verbraucher erkennbar. Die Position der Aussparung 22 des zweiten Blockierabschnitts 3B ist ebenfalls durch eine Markierung 31 erkennbar. Die Markierung 31 ist an einer radialen Außenseite des zweiten Blockierabschnitts 3B angeordnet.

Auch der Befestigungsabschnitt 4 verfügt über eine radial außen angeordnete Markierung 32. Die Markierung 32 stimmt dabei mit der umfangseitigen Winkelposition des Vorsprungs 18 überein. Der Vorsprung 18 ist jedoch auch in der vorliegenden Ausführungsform im unbetätigten Zustand an einer Außenseite der Abgabevorrichtung angeordnet, so dass seine Position durch den Verbraucher auch ohne die Markierung 32 erkennbar ist.

In der vorliegenden Einstellung des ersten Blockierabschnitts 3A, des zweiten Blockierabschnitts 3B und des Befestigungsabschnitts 4 sind die Markierungen 30, 31 und 32 auf denselben Drehwinkel relativ zur Betätigungsrichtung, bzw. zu einer gemeinsamen Drehachse der Abgabevorrichtung 1 angeordnet. Der Benutzer kann also durch ein Ausrichten der drei Markierungen 30, 31 und 32 die Abgabevorrichtung 1 in einen betätigbaren Zustand versetzen.

Die Abgabevorrichtung 1 weist weiterhin Verriegelungsmechanismen zwischen dem Betätigungsabschnitt 2, den Blockierabschnitten 3A, 3B und dem Befestigungsabschnitt 4 auf. Die Abschnitte 2, 3A, 3B und 4 sind also beispielsweise stabil verrastet, so dass ein Trennen der Blockierabschnitte 3A, 3B und des Betätigungsabschnitts 2 vom Befestigungsabschnitt 4 ohne sehr großen Kraftaufwand nicht möglich ist.

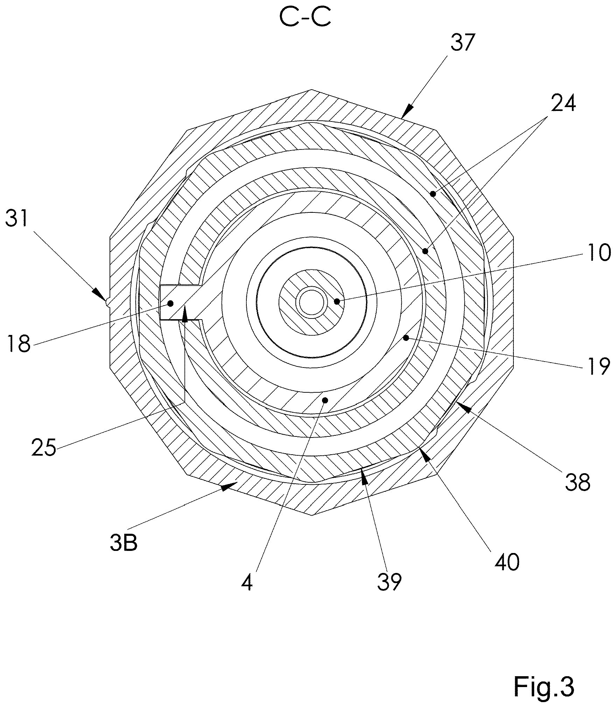

Die Fig. 2 und 3 zeigen nun Querschnitte der Abgabevorrichtung 1 entsprechend der Einstellung nach Fig. 1.

Fig. 2 zeigt eine Darstellung entlang dem Querschnitt B-B in Blickrichtung nach unten bzw. in Richtung vom Betätigungsabschnitt 2 in Richtung zum Befestigungsabschnitt 4. In dieser Darstellung lässt sich deutlicher erkennen, dass die Aussparungen 20 des ersten Blockierabschnitts 3A und die Aussparung 22 des zweiten Blockierabschnitts 3B aufeinander ausgerichtet sind. Der Vorsprung 18 ist stirnseitig von oben sichtbar. Wie klar erkennbar ist, ist die Sperrvorrichtung 17 hier so eingestellt, dass der Vorsprung 18 in die Aussparungen 20, 22 eingreifen kann. Somit ist eine Betätigung der Abgabevorrichtung 1 möglich.

In der vorliegenden Ausführungsform der Fig. 1 bis 9 weist der erste Blockierabschnitt 3A relativ zum Befestigungsabschnitt 4 eine diskrete Freigabeposition und neun diskrete Blockadepositionen auf. Dies ist dadurch realisiert, dass der Innenzylinder 21 an seiner radialen Außenseite als Zehnkant ausgebildet ist. Der Innenzylinder 21 weist also an seiner radialen Außenseite gerade Flächen 33 und Kanten 34 auf. Die Kanten 34 sind abgerundet und befinden sich umfangseitig zwischen je zwei geraden Flächen 33. Zur Ausbildung der zehn Einstellpositionen weist der zweite Blockierabschnitt 3B im vorliegenden Beispiel Erweiterungen 35 auf, die an einer radialen Innenseite des zweiten Blockierabschnitts 3B angeordnet sind. In dieser Ausführungsform sind zwei Erweiterungen 35 umfangseitig an gegenüberliegenden Seiten des zweiten Blockierabschnitts 3B angeordnet. Der erste Blockierabschnitt 3A kann nun, wenn er gegenüber dem zweiten Blockierabschnitt 3B verdreht wird, nur dann in einer Position einrasten, wenn eine Erweiterung 35 einer geraden Fläche 33 parallel gegenüberliegt. Während des Drehvorgangs zwischen erstem Blockierabschnitt 3A und zweitem Blockierabschnitt 3B überstreicht eine Kante 34, die Erweiterung 35 und wird dabei vorzugsweise elastisch verformt. Hierdurch ergibt sich ein kleiner mechanischer Widerstand der dem Anwender zeigt, wenn der zweite Blockierabschnitt 3B und der erste Blockierabschnitt 3A noch nicht in einer Position eingerastet sind.

Fig. 3 zeigt nun einen weiteren Querschnitt der Abgabevorrichtung 1 entsprechend der Einstellung nach Fig. 1 entlang dem Querschnitt C-C.

Dieser Querschnitt durchschneidet nun direkt die Markierung 31 des zweiten Blockierabschnitts 3B. Die Markierungen 30, 31 und 32 sind in der vorliegenden Ausführungsform der Abgabevorrichtung 1 als radial nach außen vorstehende Spitzen ausgeführt. Diese können beispielsweise auch von einem Verbraucher mit eingeschränkter Sehkraft ertastet werden.

Wie aus Fig. 2 ersichtlich, ist der erste Blockierabschnitt 3A an seiner radialen Außenseite mit geraden Außenflächen 36 ausgestattet. Gleichzeitig ist der zweite Blockierabschnitt 3B an seiner radialen Außenseite mit geraden Außenflächen 37 ausgestattet. Somit sind sowohl der erste Blockierabschnitt 3A als auch der zweite Blockierabschnitt 3B in der vorliegenden Ausführungsform an ihren radialen Außenseiten als Zehnkant ausgebildet. Dies erleichtert dem Verbraucher ein Verdrehen der Abschnitte, da ein Vielkant leichter zu greifen ist als ein runder Ring. Hier ist jedoch auch jede andere Art von Vieleck vorstellbar, wobei die Zahl der Kanten dann mit der Anzahl der möglichen Einstellpositionen von erstem Blockierabschnitt 3A und zweitem Blockierabschnitt 3B jeweils relativ zum Befestigungsabschnitt korrespondieren sollte.

Entsprechen Fig. 1 bis 9 ist auf den radialen Außenseiten des ersten Blockierabschnitts 3A, des zweiten Blockierabschnitts 3B und des Befestigungsabschnitt 4 jeweils nur eine Markierung 30, 31 und 32 vorgesehen. Alternativ ist es auch vorstellbar, auf jeder der geraden Außenflächen 36, 37 bzw. auch auf gegebenenfalls geraden Außenflächen des Befestigungsabschnitts 4 verschiedene Markierungen anzubringen. Vorstellbar ist beispielsweise gerade bei einem Zehnkant jeweils eine Nummerierung von null bis neun, wodurch sich beispielsweise ein Zahlenschloss ergäbe.

In Fig. 3 ist auch der Führungsring 24 noch einmal im Detail zu erkennen. Hieraus ist auch ersichtlich wie der Vorsprung 18 des Befestigungsabschnitts 4 in die Lücke 25 eingreift. Der Befestigungsabschnitt 4 ist somit mit dem Führungsring 24 drehfest verbunden. Eine Relativbewegung in Axialrichtung zwischen dem Führungsring 24 und dem Befestigungsabschnitt 4 ist aber dennoch möglich.

Fig. 3 zeigt nun auch wie die diskreten Einstellpositionen des Blockierrings 23 bzw. des zweiten Blockierabschnitts 3B erreicht werden können. Hierzu findet sich eine analoge Lösung zu den diskreten Einstellpositionen des ersten Blockierabschnitts 3A entsprechend Fig. 2. Der zweite Blockierabschnitt 3B weist an einer radialen Innenseite Erweiterungen 38 auf. Gleichzeitig umfasst der Führungsring 24 an einer radialen Außenseite gerade Fläche 39 sowie Kanten 40. Die Erweiterungen 38, die geraden Flächen 39 und die Kanten 40 wirken nun (entsprechend der geraden Flächen 33, der Kanten 34 und der Erweiterungen 35) zusammen und definieren zehn diskrete Einstellpositionen des zweiten Blockierabschnitts 3B relativ zum Führungsring 24 und damit auch zum Befestigungsabschnitt 4.

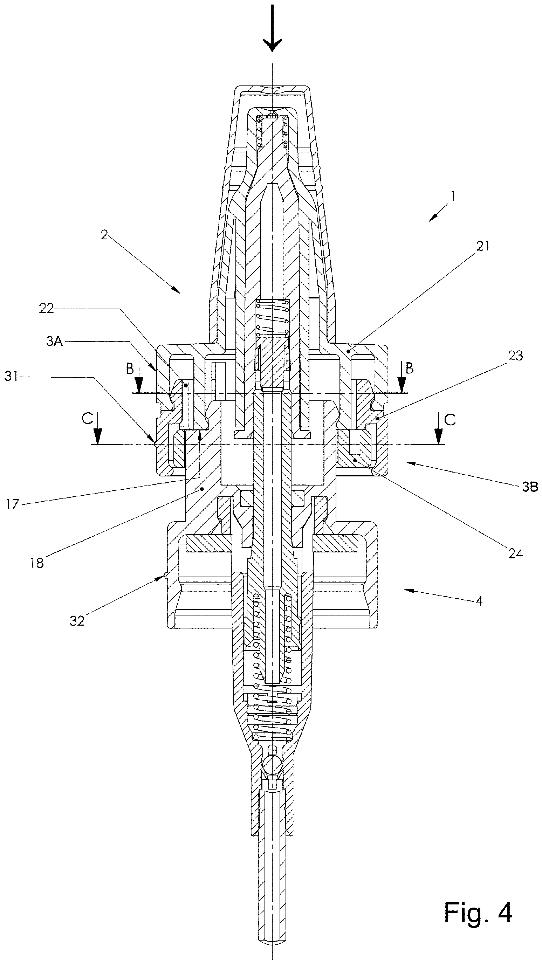

Die Fig. 4 bis 6 zeigen nun dieselbe Abgabevorrichtung entsprechend den Fig. 1 bis 3, wobei jedoch die diskrete Einstellposition des Betätigungsabschnitts 2 bzw. des ersten Blockierabschnitts 3A relativ zum Befestigungsabschnitt 4 verändert wurde.

Die Sperreinrichtung 17 ist in diesem Fall blockiert, da der Vorsprung 18 mit einer Stirnseite an der Innenhülse 21 des ersten Blockierabschnitts 3A bzw. des Betätigungsabschnitts 2 anliegt. Dementsprechend ist auch die Markierung 30 nicht auf denselben Drehwinkel wie die Markierungen 31 und 32 gedreht und in den Fig. 4 bis 6 nicht sichtbar. Die Aussparung 22 des zweiten Blockierabschnitts 3B ist zwar auf eine Freigabeposition gedreht, dies ist jedoch allein nicht ausreichend um die Abgabevorrichtung 1 in einen betätigbaren Zustand zu bringen. Vielmehr ist es notwendig, dass beide Aussparungen 20, 22 der Sperrvorrichtung 17 auf den Vorsprung 18 in Drehrichtung ausgerichtet sind.

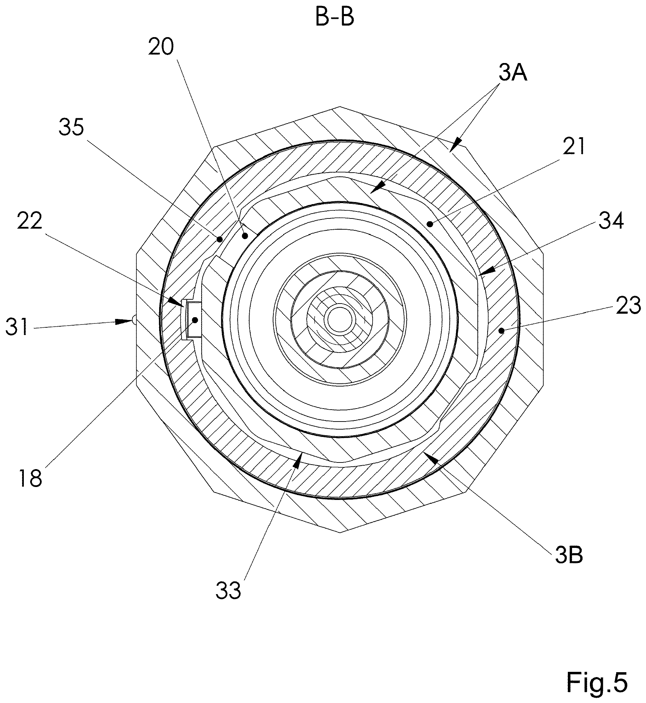

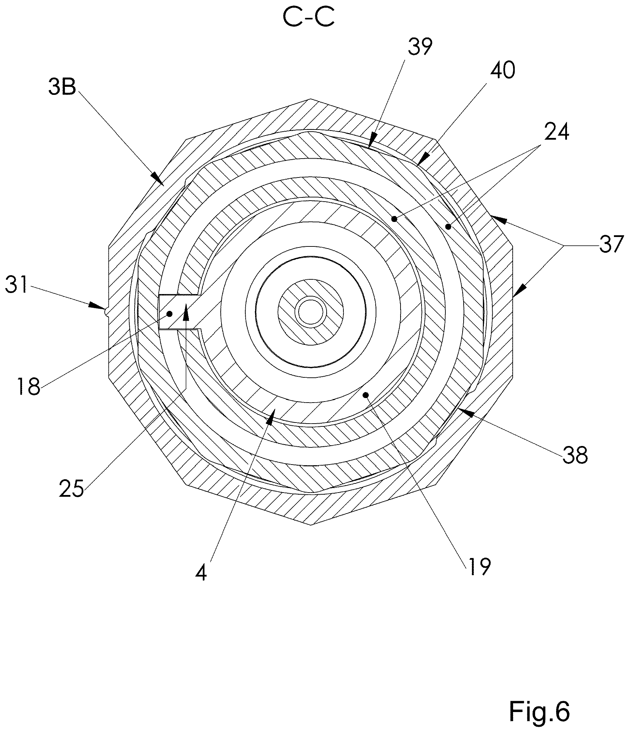

Dies lässt sich auch noch einmal in den Fig. 5 und 6 erkennen, die wiederrum zwei verschiedene Querschnitte entlang den Linien B-B und C-C darstellen. In Fig. 5 ist nun zu erkennen, dass die Aussparung 20 durch ein Verdrehen des ersten Blockierabschnitts 3B um eine diskrete Drehposition im Uhrzeigersinn gegenüber dem zweiten Blockierabschnitt 3B und dem Befestigungsabschnitt 4 verdreht wurde. Der Vorsprung 18 kann daher nicht in die Aussparung 20 eingreifen und stößt bei einem Betätigungsversuch vielmehr gegen eine Stirnseite der Innenhülse 21. Eine Betätigung der Abgabevorrichtung 1 ist somit in dieser Einstellung nicht möglich.

Fig. 6 zeigt nun einen weiteren Querschnitt der Abgabevorrichtung 1 entsprechend der Einstellung nach Fig. 4 entlang dem Querschnitt C-C.

Hier lässt sich noch einmal klar erkennen, dass der zweite Blockierabschnitt 3B sich nach wie vor in derselben Einstellung befindet wie in Fig. 1 bis 3. Dementsprechend ist die Markierung 31 des zweiten Blockierabschnitts 3B in Drehrichtung auf den Vorsprung 18 ausgerichtet.

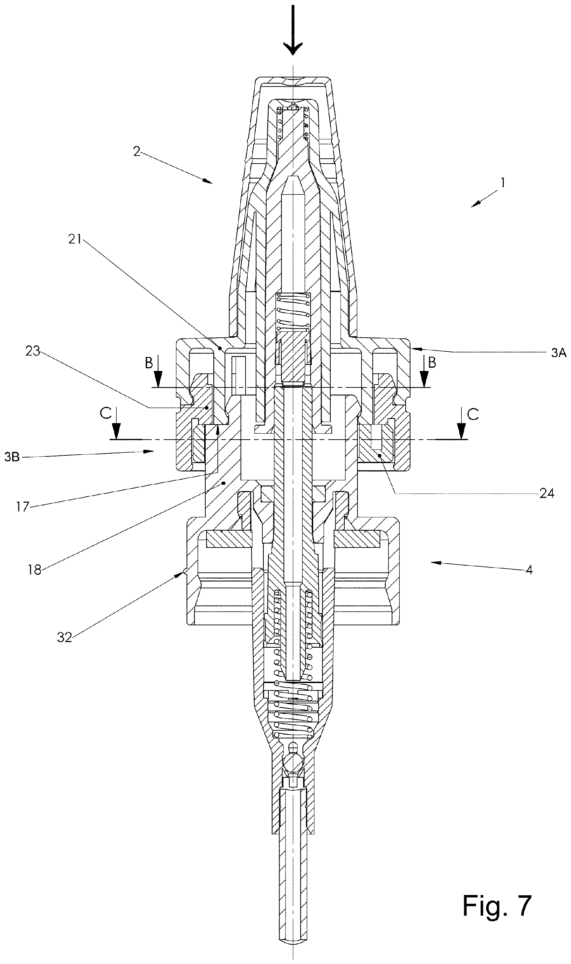

Die Fig. 7 bis 9 zeigen nun eine weitere Einstellung der Abgabevorrichtung 1. In diesem Fall befinden sich sowohl der erste Blockierabschnitt 3A als auch der zweite Blockierabschnitt 3B jeweils relativ zum Befestigungsabschnitt 4 in einer diskreten Blockadeposition. Dementsprechend liegt der Vorsprung 18 der Sperrvorrichtung stirnseitig am Blockierring 23 und an der Innenhülse 21 an. Es liegt also gewissermaßen eine doppelte Blockierung vor. Wie aus Fig. 4 bis 6 ersichtlich, reicht es jedoch bereits aus, wenn entweder der zweite Blockierabschnitt 3B oder der erste Blockierabschnitt 3A relativ zum Befestigungsabschnitt 4 in eine Blockadeposition gedreht ist. In Fig. 7 sind daher weder die Aussparungen 20, 22, noch die Markierungen 30, 31 sichtbar, da diese gegenüber dem Vorsprung 18 verdreht sind.

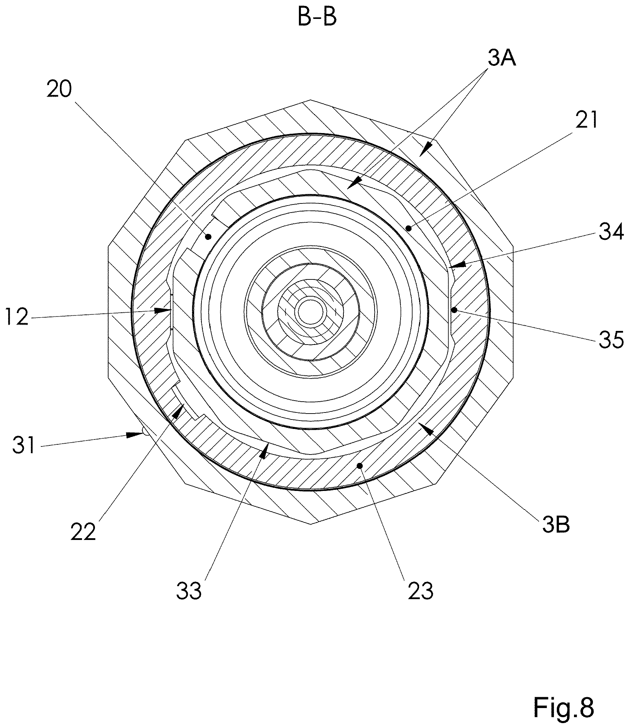

Fig. 8 zeigt nun einen Schnitt durch Fig. 7 entlang dem Querschnitt B-B. Hierbei ist ersichtlich, dass der erste Blockierabschnitt 3A gegenüber dem Vorsprung 18 und somit dem Befestigungsabschnitt 4 im Uhrzeigersinn um eine diskrete Position verdreht wurde. Gleichzeitig wurde der zweite Blockierabschnitt 3B gegenüber dem Vorsprung 18 und dem Befestigungsabschnitt 4 entgegen dem Uhrzeigersinn um eine diskrete Drehposition verdreht. Dementsprechend ist die Stirnseite des Vorsprungs 18 in dieser Darstellung auch kaum erkennbar, da sie durch die Innenhülse 21 und den Blockierring 23 verdeckt ist.

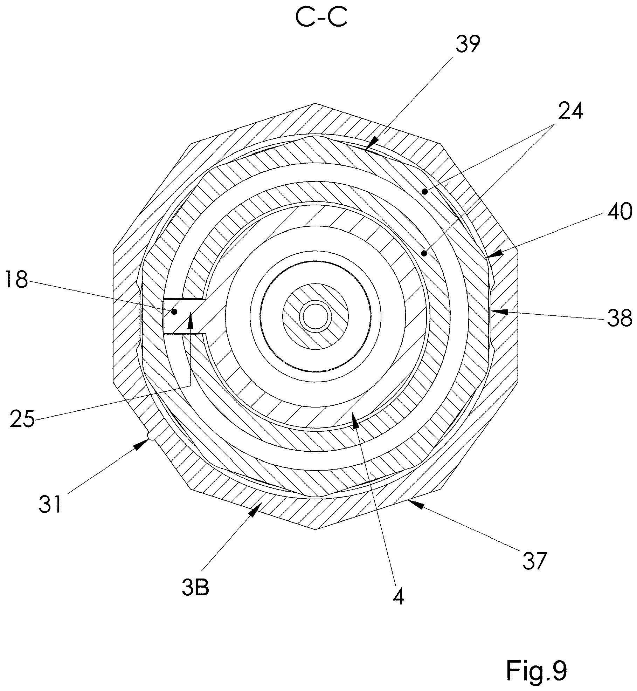

Fig. 9 zeigt nun den Querschnitt entlang dem Querschnitt C-C entsprechen Fig. 7. Hieraus ist erkennbar, dass der Vorsprung 18 sich nach wie vor an derselben Position befindet wie auch schon in den vorherigen acht Figuren. Gleichzeitig ist deutlich berkennbar, dass der zweite Blockierabschnitt 3B gegenüber dem Vorsprung 18 und dem Befestigungsabschnitt 4 entgegen dem Uhrzeigersinn um eine diskrete Position verdreht wurde.

Insgesamt ergibt sich also eine Abgabevorrichtung 1 entsprechend der Erfindung, die eine Vielzahl an blockierten relativen Einstellpositionen zwischen erstem Blockierabschnitt 3A, zweitem Blockierabschnitt 3B und Befestigungsabschnitt 4 aufweist. Gleichzeitig gibt es nur eine relative Position von erstem Blockierabschnitt 3A, zweitem Blockierabschnitt 3B und Befestigungsabschnitt 4 in der die Abgabevorrichtung 1 betätigt werden kann. Diese ist gerade in den Fig. 1 bis 3 dargestellt. Die Fig. 4 bis 6 zeigen hingegen eine einfach blockierte Einstellposition, und die Fig. 7 bis 9 eine doppelt blockierte Einstellposition der drei genannten Abschnitte.

In der dargestellten Ausführungsform gibt es insgesamt 100 relative Einstellpositionen der drei Abschnitte 3A, 3B und 4, von denen 99 entweder einfach oder doppelt blockiert sind. Die Abgabevorrichtung 1 weist daher eine effektive Kindersicherung auf und gleichzeitig wird ein ungewolltes Betätigen der Abgabevorrichtung 1 zum Beispiel während des Transports zum Endverbraucher praktisch ausgeschlossen.

## Patentansprüche

1. Abgabevorrichtung (1) für Fluide aus einem Fluidbehälter, umfassend einen Befestigungsabschnitt (4), einen ersten Blockierabschnitt (3A) und einen Betätigungsabschnitt (2), wobei die Abgabe des Fluides durch Betätigung des Betätigungsabschnitts (2) erfolgt, und wobei die Betätigung des Betätigungsabschnitts (2) durch ein Verdrehen des ersten Blockierabschnitts (3A) gegenüber dem Befestigungsabschnitt (4) blockierbar ist, wobei die Abgabevorrichtung mindestens zwei Blockierabschnitte (3A, 3B) aufweist, wobei die Betätigung des Betätigungsabschnitts (2) auch durch ein Verdrehen des zweiten Blockierabschnitts (3B) gegenüber dem Befestigungsabschnitt (4) blockierbar ist, **dadurch gekennzeichnet, dass** die Abgabevorrichtung nur in einer relativen Position von erstem Blockierabschnitt (3A), zweitem Blockierabschnitt (3B) und Befestigungsabschnitt (4) betätigt werden kann.

2. Abgabevorrichtung nach Anspruch 1, **dadurch gekennzeichnet, dass** der erste Blockierabschnitt (3A) drehfest mit dem Betätigungsabschnitt (2) verbunden ist.

3. Abgabevorrichtung nach Anspruch 1 oder 2, **dadurch gekennzeichnet, dass** der erste Blockierabschnitt (3A) relativ zum Befestigungsabschnitt (4) mindestens eine diskrete Freigabeposition und mindestens zwei diskrete Blockadepositionen aufweist.

4. Abgabevorrichtung nach einem der Ansprüche 1 bis 3, **dadurch gekennzeichnet, dass** der zweite Blockierabschnitt (3B) relativ zum Befestigungsabschnitt (4) mindestens eine diskrete Freigabeposition und mindestens zwei diskrete Blockadepositionen aufweist.

5. Abgabevorrichtung nach einem der Ansprüche 1 bis 4, **dadurch gekennzeichnet, dass** der zweite Blockierabschnitt (3B) in Betätigungsrichtung zwischen dem ersten Blockierabschnitt (3A) und dem Befestigungsabschnitt (4) angeordnet und relativ zum ersten Blockierabschnitt (3A) verdrehbar ist.

6. Abgabevorrichtung nach einem der Ansprüche 1 bis 5, **dadurch gekennzeichnet, dass** auf einer Außenseite des ersten Blockierabschnitts (3A), des zweiten Blockierabschnitts (3B) und des Befestigungsabschnitts (4) jeweils mindestens je eine Markierung (30, 31, 32) angeordnet ist.

7. Abgabevorrichtung nach einem der Ansprüche 1 bis 6, **dadurch gekennzeichnet, dass** zwischen dem ersten Blockierabschnitt (3A), dem zweiten Blockierabschnitt (3B) und dem Befestigungsabschnitt (4) mindestens eine Sperrvorrichtung (17) angeordnet ist.

8. Abgabevorrichtung nach Anspruch 7, **dadurch gekennzeichnet**, das die mindestens eine Sperrvorrichtung (17) mindestens eine Aussparung (20, 22) und mindestens einen Vorsprung (18) umfasst.

9. Abgabevorrichtung nach Anspruch 8, **dadurch gekennzeichnet, dass** die Sperrvorrichtung (17) die Betätigung des Betätigungsabschnitts (2) blockiert, es sei denn die mindestens eine Aussparung (20, 22) und der mindestens eine Vorsprung (18) sind relativ zu einer gemeinsamen Drehachse von erstem Blockierabschnitt (3A), zweitem Blockierabschnitt (3B) und Befestigungsabschnitt (4) auf denselben Drehwinkel gedreht.

10. Abgabevorrichtung nach Anspruch 8 oder 9, **dadurch gekennzeichnet, dass** der mindestens eine Vorsprung (18) in einem unbetätigten Zustand der Abgabevorrichtung (1) an einer Außenseite der Abgabevorrichtung (1) angeordnet ist.

11. Abgabevorrichtung nach einem der Ansprüche 8 bis 10, **dadurch gekennzeichnet, dass** der erste Blockierabschnitt (3A) und der zweite Blockierabschnitt (3B) mindestens je eine Aussparung (20, 22) aufweisen und der Befestigungsabschnitt (4) mindestens einen Vorsprung (18) aufweist.

12. Abgabevorrichtung nach einem der Ansprüche 1 bis 11, **dadurch gekennzeichnet, dass** der erste Blockierabschnitt (3A) und / oder der zweite Blockierabschnitt (3B) einen Blockierring (23) umfasst, der mit dem Betätigungsabschnitt (2) drehbar verbunden ist.

13. Abgabevorrichtung nach einem der Ansprüche 1 bis 12, **dadurch gekennzeichnet, dass** die Abgabevorrichtung (1) einen Führungsring (24) aufweist, der gegenüber dem Befestigungsabschnitt (4) in Betätigungsrichtung verschiebbar ist.

14. Abgabevorrichtung nach Anspruch 13, **dadurch gekennzeichnet, dass** der Führungsring (24) gegenüber dem Befestigungsabschnitt (4) drehfest angeordnet ist.

## Claims

1. A dispensing device (1) for fluids from a fluid container, comprising an attachment section (4), a first blocking section (3A) and an actuating section (2), wherein a dispensing of the fluid results from actuation of the actuating section (2), and wherein the actuation of the actuation section (2) is blockable by a rotation of the first blocking section (3A) relative to the attachment section (4), wherein the dispensing device comprises at least two blocking sections (3A, 3B), wherein the actuation of the actuating section (2) is also blockable by a rotation of the second blocking section (3B) relative to the attachment section (4), **characterized in that** the dispensing device is actuatable only in one relative position of the first blocking section (3A), the second blocking section (3B) and the attachment section (4).

2. Dispensing device according to claim 1, **characterized in that** the first blocking section (3A) is non-rotatably connected to the actuating section (2).

3. Dispensing device according to claim 1 or 2, **characterized in that** the first blocking section (3A) comprises at least one discreet release position and at least two discreet blocking positions relative to the attachment section (4).

4. Dispensing device according to any of claims 1 to 3, **characterized in that** the second blocking section (3B) comprises at least one discreet release position and at least two discreet blocking positions relative to the attachment section (4).

5. Dispensing device according to any of claims 1 to 4, **characterized in that** the second blocking section (3B), with regard to an actuation direction, is arranged between the first blocking section (3A) and the attachment section (4) and is rotatable relative to the first blocking section (3A).

6. Dispensing device according to any of claims 1 to 5, **characterized in that** at least one marking (30, 31, 32) each is arranged respectively on an outside of the first blocking section (3A), the second blocking section (3B) and the attachment section (4).

7. Dispensing device according to any of claims 1 to 6, **characterized in that** at least one locking device (17) being arranged between the first blocking section (3A), the second blocking section (3B) and the attachment section (4).

8. Dispensing device according to claim 7, **characterized in that** the at least one locking device (17) comprises at least one recess (20, 22) and at least one projection (18).

9. Dispensing device according to claim 8, **characterized in that** the locking device (17) blocks the actuation of the actuating section (2), unless the at least one recess (20, 22) and the at least one projection (18) are rotated to the same angle of rotation relative to a common rotational axis of the first blocking section (3A), the second blocking section (3B) and the attachment section (4).

10. Dispensing device according to claim 8 or 9, **characterized in that** in an unactuated state of the dispensing device (1), the at least one projection (18) is arranged on an outside of the dispensing device (1).

11. Dispensing device according to any of claims 8 to 10, **characterized in that** the first blocking section (3A) and the second blocking section (3B) comprise at least one recess (20, 22) each and the attachment section (4) comprises at least one projection (18).

12. Dispensing device according to any of claims 1 to 11, **characterized in that** the first blocking section (3A) and/or the second blocking section (3B) comprises a blocking ring (23) that is rotatably connected to the actuating section (4).

13. Dispensing device according to any of claims 1 to 12, **characterized in that** the dispensing device (1) comprises a guide ring (24) that is displaceable, with respect to the attachment section (4), in an actuating direction.

14. Dispensing device according to claim 13, **characterized in that** the guide ring (24) is arranged in a non-rotatable manner with respect to the attachment section (4).

## Revendications

1. Dispositif de distribution (1) pour des fluides provenant d'un contenant de fluide, comprenant un segment de fixation (4), un premier segment de blocage (3A) et un segment d'actionnement (2), dans lequel la distribution du fluide est effectuée par l'actionnement du segment d'actionnement (2), et l'actionnement du segment d'actionnement (2) peut être bloqué par une rotation du premier segment de blocage (3A) par rapport au segment de fixation (4), dans lequel le dispositif de distribution présente au moins deux segments de blocage (3A, 3B), dans lequel l'actionnement du segment d'actionnement (2) peut être bloqué également par une rotation du deuxième segment de blocage (3B) par rapport au segment de fixation (4), **caractérisé en ce que** le dispositif de distribution peut être actionné seulement dans une position relative par le premier segment de blocage (3A), le deuxième segment de blocage (3B) et le segment de fixation (4).

2. Dispositif de distribution selon la revendication 1, **caractérisé en ce que** le premier segment de blocage (3A) est relié de manière solidaire en rotation au segment d'actionnement (2).

3. Dispositif de distribution selon la revendication 1 ou 2, **caractérisé en ce que** le premier segment de blocage (3A) présente, par rapport au segment de fixation (4), au moins une position de déblocage discrète et au moins deux positions de blocage discrètes.

4. Dispositif de distribution selon l'une quelconque des revendications 1 à 3, **caractérisé en ce que** le deuxième segment de blocage (3B) présente, par rapport au segment de fixation (4), au moins une position de déblocage discrète et au moins deux positions de blocage discrètes.

5. Dispositif de distribution selon l'une quelconque des revendications 1 à 4, **caractérisé en ce que** le deuxième segment de blocage (3B) est disposé dans la direction d'actionnement entre le premier segment de blocage (3A) et le segment de fixation (4) et peut être tourné par rapport au premier segment de blocage (3A).

6. Dispositif de distribution selon l'une quelconque des revendications 1 à 5, **caractérisé en ce que** respectivement au moins un repère (30, 31, 32) est disposé sur un côté extérieur du premier segment de blocage (3A), du deuxième segment de blocage (3B) et du segment de fixation (4).

7. Dispositif de distribution selon l'une quelconque des revendications 1 à 6, **caractérisé en ce qu'**au moins un dispositif de fermeture (17) est disposé entre le premier segment de blocage (3A), le deuxième segment de blocage (3B) et le segment de fixation (4).

8. Dispositif de distribution selon la revendication 7, **caractérisé en ce que** l'au moins un dispositif de fermeture (17) comprend au moins un évidement (20, 22) et au moins une partie faisant saillie (18).

9. Dispositif de distribution selon la revendication 8, **caractérisé en ce que** le dispositif de fermeture (17) bloque l'actionnement du segment d'actionnement (2), à moins que l'au moins un évidement (20, 22) et l'au moins une partie faisant saillie (18) soient tournés sur le même angle de rotation par rapport à un axe de rotation commun par le premier segment de blocage (3A), par le deuxième segment de blocage (3B) et par le segment de fixation (4).

10. Dispositif de distribution selon la revendication 8 ou 9, **caractérisé en ce que** l'au moins une partie faisant saillie (18) est disposée, dans un état non actionné du dispositif de distribution (1), au niveau d'un côté extérieur du dispositif de distribution (1).

11. Dispositif de distribution selon l'une quelconque des revendications 8 à 10, **caractérisé en ce que** le premier segment de blocage (3A) et le deuxième segment de blocage (3B) présentent au moins respectivement un évidement (20, 22), et **en ce que** le segment de fixation (4) présente au moins une partie faisant saillie (18).

12. Dispositif de distribution selon l'une quelconque des revendications 1 à 11, **caractérisé en ce que** le premier segment de blocage (3A) et/ou le deuxième segment de blocage (3B) comprennent un anneau de blocage (23), qui est relié de manière à pouvoir tourner autour segment d'actionnement (2).

13. Dispositif de distribution selon l'une quelconque des revendications 1 à 12, **caractérisé en ce que** le dispositif de distribution (1) présente un anneau de guidage (24), qui peut être coulissé dans la direction d'actionnement par rapport au segment de fixation (4).

14. Dispositif de distribution selon la revendication 13, **caractérisé en ce que** l'anneau de guidage (24) est disposé de manière solidaire en rotation par rapport au segment de fixation (4).
